# EUROPEAN PATENT SPECIFICATION

(11) **EP 0 836 449 B1**
(45) Date of publication and mention of the grant of the patent: **27.08.2003**
(21) Application number: 96918203.9
(22) Date of filing: 05.06.1996
(51) Int. Cl.: A61F 2/06

(54) **PROSTHETIC GRAFT FOR ANEURYSM REPAIR**
TRANSPLANTAT ZUM AUSBESSERN EINES ANEURYSMAS
GREFFE PROSTHETIQUE DE REPARATION D'UN ANEVRISME

(30) Priority: 06.06.1995 US 472700
(43) Date of publication of application: 22.04.1998
(73) Proprietor: Endotex Interventional Systems, Inc., Menlo Park, CA 94025 (US)
(72) Inventor: ORTH, Geoffrey, A., El Granada, CA 94018 (US)
(74) Representative: VOSSIUS & PARTNER
(86) International application number: US9609149
(87) International publication number: WO96039104

(56) References cited:
- EP-A- 0 321 912
- EP-A- 0 539 237
- EP-A- 0 556 850
- EP-A- 0 637 454

## Description

This invention relates to the delivery and placement of vascular grafts, and in particular, to a graft delivery system for the repair of abdominal aortic aneurysms.

An aneurysm is a sac resulting from abnormal dilation of the artery wall and is often associated with arteriosclerotic disease. Unless treated, an aneurysm can rupture, leading to severe and often fatal hemorrhaging. Treating an aortic aneurysm generally involves transplanting a prosthetic graft to bridge the affected section of the aorta. Surgical implantation of the graft is possible but this treatment causes considerable trauma, results in high mortality and morbidity and, even when completely successful, requires a lengthy recuperation period. Due to the difficulty of the operation, direct surgical replacement is even less attractive when it must be performed on an emergency basis after the aneurysm has ruptured.

A less invasive alternative involves the use of a catheter to effect intraluminal delivery of a graft. Prior art graft delivery systems, such as disclosed in EP 0 461 791 Al (Barone et al.), employ a graft with expandable portions that anchor the graft in the aorta. Often, the systems use an inflatable balloon on the delivery catheter to expand the anchoring portion of the graft as disclosed in United States Pat. No. 5,275,622 (Lazarus et al.) This latter example requires the use of a bulky capsule to store the graft and a complicated pushrod system to deploy the graft. EP-A-0 539 237 describes a graft delivery system wherein a central control means is employed to maintain the axial position of a graft during deployment of self-expanding spring assemblies. EP-A-o 637 454 describes a graft delivery system as specified in the preambles of claims 1 and 4, wherein a ballon is used to firmly implant attachment means coupled to the graft within a vessel.

Although the referenced prior art systems and others employ many different stent and graft configurations, none are completely satisfactory. The principle limitation of the prior art systems is their size. They typically require a delivery catheter having a diameter of approximately 24-28 French (8 to 9.3 mm). Although it is desirable to introduce grafts through the femoral artery, its inner diameter is only about 4 to 6 mm. Thus, the size of the prior art devices restricts them to introduction through upper femoral sites, where access may be difficult. Accordingly, there is a need for a graft system capable of introduction through a smaller opening while maintaining the ability to reliably and securely deploy the graft.

The success of a percutaneous vessel repair depends in large part on getting the graft to the location of the vasculature in need of repair and deploying the graft effectively. Another difficulty associated with prior art graft deployment systems is blood flow-by which occurs when blood can pass between the graft and the patient's vessel, bypassing the graft. There is a need for a graft system which minimizes or prevents flow-by.

### SUMMARY OF THE INVENTION

This invention provides a graft delivery system for aneurysm repair as specified in claims 1 and 4, which system generally comprises a prosthetic graft with means to draw a distal extremity of the graft over a portion of an expandable anchoring member. For deployment, the graft and the anchoring member are radially compressed and positioned adjacent one another coaxially on a delivery catheter with essentially no overlap between the distal end of the graft and the proximal end of the anchoring member. The drawing means is connected to the distal extremity of the graft so that when tension is applied to the graft by the drawing means, the distal extremity of the graft is caused to overlap a proximal portion of the anchoring member. Subsequent expansion of the anchoring member seals the overlapped graft against the vessel wall to minimize blood flow-by and secures the graft in the vessel. Since the graft and anchoring member do not overlap when loaded on the catheter prior to deployment, the system maintains an overall low profile.

In a preferred embodiment, the drawing means comprises at least one and preferably four pull strands attached to the distal end of the graft. The pull strands extend outside the distal end of the catheter, are threaded through an opening at the distal end of the delivery catheter and extend through a lumen in the catheter to the proximal end. After the graft is properly positioned within a patient's vasculature, tension is applied to the pull strands to cause the graft to slide distally along the catheter until the distal end of the graft extends over the proximal end of the anchoring member. Subsequent expansion of the anchoring member expands the graft, anchors it against the vessel wall and seals the graft against the vessel wall to minimize blood flow-by. Securing means may be advanced up the pull strand to abut the anchoring member and further secure the graft to the anchoring member.

The graft may also comprise means to guide a second expandable anchoring member to the proximal end of the graft. The guiding means are connected to a proximal extremity of the graft. In one embodiment, the guiding means comprises at least one and preferably four guide strands. After the distal end of the graft is anchored within the patient's vasculature, the guide strands are laced through the second anchoring member. The anchoring member can then be advanced up the guide strands until it is adjacent the proximal end of the graft. The graft overlaps the second anchoring member and expansion of the anchoring member secures the proximal end of the graft. Preferably, securing means secure the proximal end of the graft to the second anchoring member as described above regarding the drawing means.

### BRIEF DESCRIPTION OF THE DRAWINGS

FIG. **1** is a sectional elevation of a delivery catheter and graft system embodying features of this invention.
FIG. **2** is an elevational view of a distal portion of a delivery catheter and graft system of the invention comprising a self-expanding stent.
FIG. **3** shows an embodiment of the invention comprising pull strand securing means.
FIG. **4** illustrates an embodiment of the invention comprising guiding means and downstream graft anchoring.

### DETAILED DESCRIPTION OF THE INVENTION

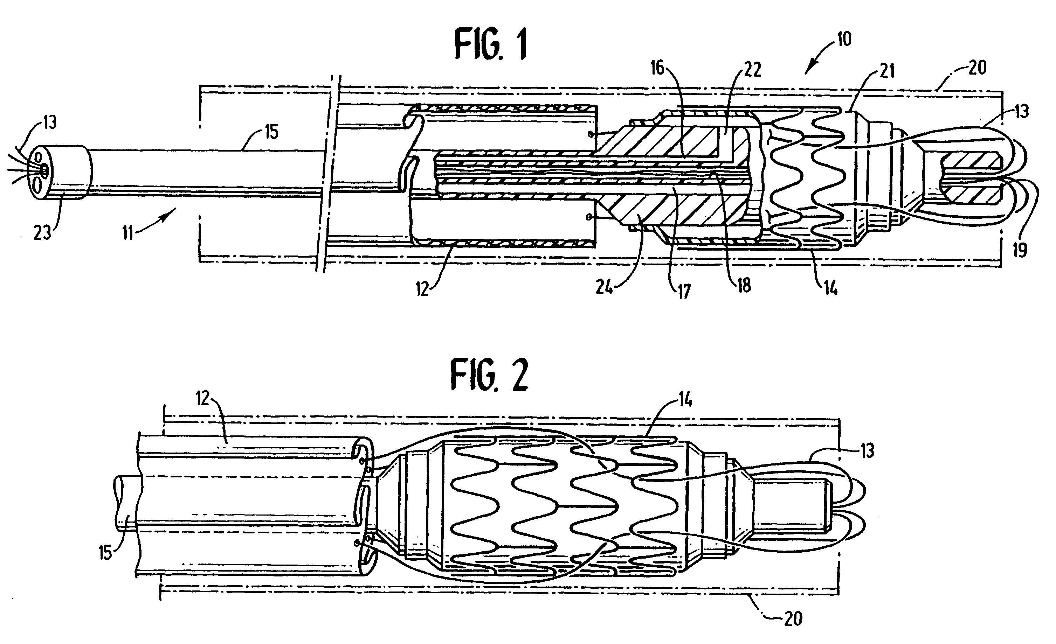

FIG. **1** is a elevational view, in section, illustrating a catheter system **10** embodying features of the invention which generally comprises a catheter **11** with an aortic graft **12** having drawing means, a plurality of pull strands **13,** attached to the distal end and an expandable anchoring member, stent **14,** loaded for delivery. The catheter **11** comprises a flexible catheter shaft **15** with proximal and distal ends, an inflation lumen **16,** a guidewire lumen **17** and a pull strands lumen **18.** Pull strands **13** extend from graft **12,** enter the pull strands lumen **18** through opening **19** at the distal end of catheter **11** and exit from the proximal end of catheter **11.** Preferably, pull strands **13** may be laced through stent **14** at a point corresponding to the amount of overlap desired. In a preferred embodiment, graft **12** overlaps about half the length of stent **14** prior to expansion. A thin-walled retractable sheath **20** is slidably disposed over the catheter **11** and configured so that it covers the stent **14** and graft **13** during introduction and placement of the catheter system **10** and may be withdrawn once they are in an appropriate position within the patient. In the embodiment of FIG. **1**, the catheter **11** includes an inflatable balloon **21** in fluid communication with the inflation lumen **15** through inflation passage **22** which is configured to expand stent **14**. The proximal end of catheter shaft **15** can have a cap **23** bonded to the end to provide access to inflation lumen **16,** guidewire lumen **17** and pull strands lumen **18.** The configuration of cap **23** allows it to mate in a conventional manner with a multi-arm adaptor (not shown) that supplies inflation fluid and allows guidewire control and manipulation of the pull strands. Cap **23** is preferably formed from metal, although other materials such as plastics are suitable. The diameter of cap **23** allows the compressed graft **12** and stent **14** to be threaded over the proximal end of catheter shaft **14**. In a preferred embodiment, catheter shaft **15** has an increased diameter delivery base **24** under balloon **21.** The delivery base **24** provides support for the expansion of stent **14** while allowing the remainder of the catheter shaft **15** to have a relatively small diameter. Further details regarding the design of suitable catheters as well as graft and stent materials may be found in the application WO 96/14808.

Preferably, the stent **14** is a Bronco® stent, available from Advanced Cardiovascular Systems, Inc. of Santa Clara, CA. Alternatively, stent **14** may be self-expanding as shown in FIG. **2**. For example, stent **14** may be constructed from shape-memory materials causing it to revert to its expanded shape at body temperatures. In such embodiments, thin-walled sheath **20** would restrain stent **14** until properly positioned. Other suitable means for expanding the stent may be used.

The effective wall thickness of stent **14** does not change between the pre-expanded and expanded states. On the other hand, the graft **12** bunches when compressed to conform to the catheter shaft **15** and forms overlaps and pleats, effectively increasing its wall thickness. Stent **14** and graft **12** are coaxially threaded over the catheter **11;** the relatively thick compressed graft **12** is positioned solely over the relatively small diameter catheter shaft **15** while the stent **14** receives the increased support for expansion provided by larger diameter delivery base **24**. The lack of overlap between the graft **12** and the stent **14** when loaded for deployment on catheter shaft **15** saves approximately 1-3 mm from the diameter of system **10** over conventional systems. Accordingly, the delivery catheters of this invention have an insertion diameter of less than 7 mm and preferably about 6 mm. This allows system **10** to present a small outer diameter to facilitate introduction into the body, preferably through the femoral artery.

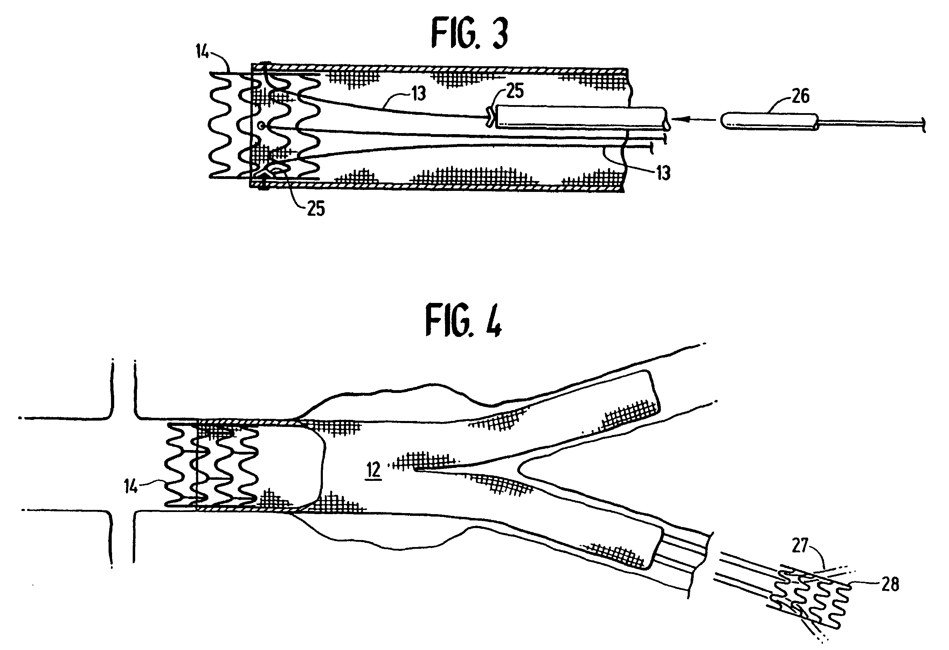

In embodiments where pull strands **13** are laced through stent **14,** securing means **25** may be employed to further anchor the graft **12** as shown in FIG. **3**. The securing means **25** thread over pull strands **13** and are configured so that they will not pass through the framework of stent **14**. In one embodiment, securing means **25** comprise one-way frictional sliding washers. Once graft **12** and stent **14** are deployed and catheter **11** removed, a simple catheter **26** is used to push securing means **25** up pull strands **13** until they are tightly seated against stent **14.** This secures pull strands **13** to stent **14** and further anchors the graft **12.**

FIG. 4 illustrates an embodiment of the invention which comprises guiding means, guide strands **27**, attached to the proximal end of graft **12.** Guide strands **27** are similar in configuration to pull strands **13.** In use, the graft's distal end (or ends in the case of a bifurcated graft) **12** may be anchored by lacing guide strands **27** through a second anchoring member, stent **28,** and advancing the stent **28** up the guide strands until it is at least immediately adjacent to the proximal end of graft **12.** Once positioned, the anchoring member **28** is expanded to secure the proximal end of graft **12** and maintain the graft's patency. Securing means **25** may be used to further anchor the graft as described above. In some embodiments, the guide strands **27** are attached to the interior of graft **12** at a point inset from the proximal end to allow that proximal portion of graft **12** to overlap a distal portion of stent **28** such that expansion of stent **28** will sandwich the proximal end of the graft against the patient's vessel wall.

In general, anchoring members may be formed from any suitable material, including tantalum, stainless steel, other metals and polymers and when employing a self-expanding anchoring member, shape-memory metals such as NiTi alloys. The anchoring member may also be coated with a polymer or seeded with endothelial cells to further inhibit thrombosis. Generally, the anchoring member is formed in its pre-expanded state. Once it is positioned with the graft over the catheter shaft **15,** the anchoring member may be crimped down to a slightly smaller inner diameter onto the means for expanding the anchoring member to secure the assembly during introduction and to further reduce the delivery diameter. Anchoring member configurations are suitable so long as they are self supporting within the aortic passageway and provide suitable means for anchoring the graft.

The grafts of this invention are intact tubes, preferably constructed of a synthetic yarn, monofilament or multifilament, formed from materials such as polyesters (including Dacron®), polytetrafluoroethylene (PTFE), polyurethane or nylon. Dacron® in particular, a multifilament composed of polyethylene terephthalate (PET), has been shown to be suitable and may promote formation of intima. The synthetic material may be woven or knit. The woven grafts are generally stronger and less porous while knit grafts are softer and more porous. Additionally, the surface of the synthetic material may be texturized and woven or knit to form a velour surface which generally facilitates growth of tissue from the surrounding lumen through the velour loops to help secure the graft. If desirable, the graft may be bifurcated.

The catheter **11** may be formed from any suitably flexible material, such as pseudoelastic metals (*e.g*. NiTi alloys) and a wide range of conventional polymers. Preferably, the catheter **11** is formed from an extrudable polymer such as polyethylene (PE).

The inflatable balloon **21** may be a relatively inelastic inflatable balloon to provide the degree of expansion control and durability necessary to effectively expand and anchor the anchoring member. The balloon may be formed from any suitable material such as PE, polyethylene terephthalate (PET) or nylon or other polyamide.

Pull strands **13** and guide strands **27** may comprise any suitable material having sufficient flexibility to easily thread through the anchoring means and the catheter while having sufficient strength to pull the graft over the stent. Suitable materials include plastics and metals. In some embodiments, pull strands **13** and guide strands **27** may comprise integral parts of the graft **12.** It may be desirable to form all or a portion of pull strands **13** and guide strands **27** from a bioabsorbable material. It is preferable to provide the graft **12** with four pull strands **13** and four guiding strands **27,** each radially spaced around the respective end of the graft.

The use of the catheter system **10** generally follows conventional procedures. In particular, a guidewire (not shown) is backloaded into guidewire receiving lumen **17** of the catheter **11** with sheath **20** extending over the compressed and loaded graft **12** and stent **14,** as shown in phantom. The catheter system **11** and guidewire are percutaneously introduced by means of conventional cut down techniques in the patient's arterial system, generally through the femoral artery. The guidewire is advanced out of the delivery catheter **11** and up the aorta via fluoroscopic imaging until it crosses the aneurysm. Then the catheter **11** is advanced over the guidewire until the stent **14** is positioned within a healthy region of the aorta upstream from the aneurysm. The sheath **20** is retracted to expose the stent **14** and the graft **12.** Pull strands **13** are pulled to draw the distal end of the graft a desired amount over the proximal end of the stent **14,** preferably half the length of the stent. Then, balloon **21** is inflated to expand stent **14** to sandwich the distal end of graft **12** between the stent **14** and the aortic wall, thus anchoring it. The balloon **21** is deflated and the catheter **11** is removed, leaving the expanded anchoring member and graft in place. To further secure the graft, securing means **25** may be advanced up pull strands **13** until they abut the stent **14.** Once securing means **25,** if any, are positioned, all excess of the pull strands **13** may be cut.

Once deployed, the proximal end or ends of graft **12** may be anchored by advancing another anchoring member **28** along guide strands **27.** Securing means **25** may also be employed to further anchor the proximal end of graft **12** to anchoring member **28.**

## Claims

1. A graft delivery system (10) comprising an elongated catheter (11) with proximal and distal ends, and a means for expanding (21) an expandable anchoring member (14) on a distal portion of the catheter (11); an expandable anchoring member (14) with proximal and distal ends mounted on the catheter (11), and an aortic graft (12) in a radially compressed state with proximal and distal ends mounted on the catheter (11) proximally adjacent to the anchoring member (14), **characterized in that**
the anchor member (14) is mounted on the expanding means (21) and the distal end of the graft (12) does not overlap the proximal end of the anchor member (14); and
drawing means (13) secured to the distal end of the graft (12) and being arranged and passing in such a way through the graft delivery system (10) that upon traction thereon from the proximal end of the graft delivery system (10) the distal end of the graft (12) is pulled over the proximal end of the anchoring member (14).

2. The graft delivery system (10) of claim 1 wherein the means for expanding (21) an anchoring member (14) comprises an inflatable member (21).

3. The graft delivery system (10) of claim 1 or 2 including an inflation lumen (16) extending through the catheter (11) in fluid communication with the interior of the inflatable member (21).

4. A graft delivery system (10) comprising an elongated catheter (11) with proximal and distal ends, a self-expanding anchoring member (14) with proximal and distal ends mounted on a distal portion of the catheter (11), an aortic graft (12) in a radially compressed state with proximal and distal ends and mounted on the catheter (11) proximally adjacent to the anchoring member (14); **characterized in that**
the distal end of the graft (12) does not overlap the proximal end of the anchor member (14); and the graft delivery system (10) includes drawing means (13) secured to the distal end of the graft (12) and being arranged and passing in such a way through the graft delivery system (10) that upon traction thereon from the proximal end of the graft delivery system (10) the distal end of the graft (12) is pulled over the proximal end of the anchoring member (14).

5. The graft delivery system of claim 4 wherein the self-expanding anchoring member (14) is formed from shape-memory material.

6. The graft delivery system of claim 5 wherein the anchoring member (14) is formed from a NiTi alloy.

7. The graft delivery system (10) of any of claims 1 to 6 wherein the catheter (11) further comprises a lumen (18) with an opening (19) at the distal end and extending to the proximal end and the drawing means (13) comprises at least one pull strand (13) extending from the distal end of the graft (12) through the opening (19) at the distal end of the catheter (11) to the proximal end of the catheter (11).

8. The graft delivery system (10) of claim 7 wherein the pull strands (13) are laced through the anchoring member (14).

9. The graft delivery system (10) of any of claims 1 to 8 wherein the graft delivery system (10) has an outer diameter of less than about 7 mm.

10. The graft delivery system (10) of any of claims 1 to 9 wherein the anchoring member (14) comprises a stent (14).

11. The graft delivery system (10) of any of claims 1 to 10 wherein the graft (12) further comprises guiding means (27) extending from the proximal end of the graft (12).

12. The graft delivery system (10) of claim 11 wherein the guiding means (27) comprise at least one guiding strand (27).

13. The graft delivery system (10) of claim 7 comprising a catheter (26) for disposing securing means (25) on the at least one pull strand to secure the graft (12) to the anchoring member (14).

14. The graft delivery system (10) of any of claims 1 to 13 wherein the graft (12) is a bifurcated graft (12).

## Patentansprüche

1. Transplantatzuführungssystem (10) mit einem langgestreckten Katheter (11) mit einem proximalen und einem distalen Ende und einer Einrichtung (21) zum Expandieren eines expandierbaren Verankerungsteils (14) an einem distalen Abschnitt des Katheters (11); einem expandierbaren Verankerungsteil (14) mit einem proximalen und einem distalen Ende, das am Katheter (11) angeordnet ist, und einem Aortentransplantat (12) in einem radial zusammengedrückten Zustand mit einem proximalen und einem distalen Ende, das proximal angrenzend an das Verankerungsteil (14) auf dem Katheter (11) angeordnet ist, **dadurch gekennzeichnet, daß**
das Ankerteil (14) auf der Expansionseinrichtung (21) angeordnet ist und das distale Ende des Transplantats (12) das proximale Ende des Ankerteils (14) nicht überlappt; und
eine Zugeinrichtung (13), die am distalen Ende des Transplantats (12) fest angeordnet ist und so eingerichtet und durch das Transplantatzuführungssystem (10) hindurchgeführt ist, daß beim Ziehen an dieser vom proximalen Ende des Transplantatzuführungssystems (10) das distale Ende des Transplantats (12) über das proximale Ende des Verankerungsteils (14) gezogen wird.

2. Transplantatzuführungssystem (10) nach Anspruch 1, wobei die Einrichtung (21) zum Expandieren eines Verankerungsteils (14) ein aufblasbares Teil (21) aufweist.

3. Transplantatzuführungssystem (10) nach Anspruch 1 oder 2, mit einem Einströmlumen (16), das sich in Fluidkommunikation mit dem Inneren des aufblasbaren Teils (21) durch den Katheter (11) erstreckt.

4. Transplantatzuführungssystem (10) mit einem langgestreckten Katheter (11) mit einem proximalen und einem distalen Ende, einem selbstexpandierenden Verankerungsteil (14) mit einem proximalen und einem distalen Ende, das an einem distalen Abschnitt des Katheters (11) angeordnet ist, einem Aortentransplantat (12) in einem radial zusammengedrückten Zustand mit einem proximalen und einem distalen Ende, das proximal angrenzend an das Verankerungsteil (14) auf dem Katheter (11) angeordnet ist; **dadurch gekennzeichnet, daß**
das distale Ende des Transplantats (12) das proximale Ende des Ankerteils (14) nicht überlappt; und das Transplantatzuführungssystem (10) eine Zugeinrichtung (13) aufweist, die am distalen Ende des Transplantats (12) fest angeordnet und so eingerichtet ist und durch das Transplantatzuführungssystem (10) hindurchgeführt ist, daß beim Ziehen an dieser vom proximalen Ende des Transplantatzuführungssystem (10) das distale Ende des Transplantats (12) über das proximale Ende des Verankerungsteils (14) gezogen wird.

5. Transplantatzuführungssystem nach Anspruch 4, wobei das selbstexpandierende Verankerungsteil (14) aus einem Memory-Material besteht.

6. Transplantatzuführungssystem nach Anspruch 5, wobei das Verankerungsteil (14) aus einer NiTi-Legierung besteht.

7. Transplantatzuführungssystem (10) nach einem der Ansprüche 1 bis 6, wobei der Katheter (11) ferner ein Lumen (18) aufweist, das eine Öffnung (19) am distalen Ende hat und sich zum proximalen Ende erstreckt, und die Zugeinrichtung (13) mindestens einen Zugfaden (13) aufweist, der sich vom distalen Ende des Transplantats (12) durch die Öffnung (19) am distalen Ende des Katheters (11) zum proximalen Ende des Katheters (11) erstreckt.

8. Transplantatzuführungssystem (10) nach Anspruch 7, wobei die Zugfäden (13) durch das Verankerungsteil (14) gefädelt sind.

9. Transplantatzuführungssystem (10) nach einem der Ansprüche 1 bis 8, wobei das Transplantatzuführungssystem (10) einen Außendurchmesser von weniger als 7 mm hat.

10. Transplantatzuführungssystem (10) nach einem der Ansprüche 1 bis 9, wobei das Verankerungsteil (14) einen Stent (14) aufweist.

11. Transplantatzuführungssystem (10) nach einem der Ansprüche 1 bis 10, wobei das Transplantat (12) ferner eine Führungseinrichtung (27) aufweist, die sich vom proximalen Ende des Transplantats (12) erstreckt.

12. Transplantatzuführungssystem (10) nach Anspruch 11, wobei die Führungseinrichtung (27) mindestens einen Führungsfaden (27) aufweist.

13. Transplantatzuführungssystem (10) nach Anspruch 7 mit einem Katheter (26) zum Anordnen einer Sicherungseinrichtung (25) an den mindestens einen Zugfaden, um das Transplantat (12) am Verankerungsteil (14) fest anzuordnen.

14. Transplantatzuführungssystem (10) nach einem der Ansprüche 1 bis 13, wobei das Transplantat (12) ein Gabeltransplantat (12) ist.

## Revendications

1. Système d'alimentation en greffe (10), comportant un cathéter allongé (11) ayant des extrémités proximale et distale, et des moyens pour étendre (21) un élément d'ancrage pouvant être étendu (14) sur une partie distale du cathéter (11), un élément d'ancrage pouvant être étendu (14) muni d'extrémités proximale et distale montées sur le cathéter (11), et une greffe aortique (12) dans un état comprimé radialement ayant des extrémités proximale et distale et montée sur le cathéter (11) en étant adjacente de manière proximale à l'élément d'ancrage (14), **caractérisé en ce que**
l'élément d'ancrage (14) est monté sur les moyens d'extension (21) et l'extrémité distale de la greffe (12) ne recouvre pas l'extrémité proximale de l'élément d'ancrage (14), et
des moyens d'extraction (13) fixés sur l'extrémité distale de la greffe (12) et étant agencés pour passer d'une manière telle à travers le système d'alimentation en greffe (10) que, lors d'une traction sur ceux-ci à partir de l'extrémité proximale du système d'alimentation en greffe (10), l'extrémité distale de la greffe (12) est retirée de l'extrémité proximale de l'élément d'ancrage (14).

2. Système d'alimentation en greffe (10) selon la revendication 1, dans lequel les moyens pour étendre (21) un élément d'ancrage (14) comportent un élément gonflable (21).

3. Système d'alimentation en greffe (10) selon la revendication 1 ou 2, comportant une ouverture de gonflage (16) s'étendant à travers le cathéter (11) dans une communication de fluide avec la partie intérieure de l'élément gonflable (21).

4. Système d'alimentation en greffe (10), comportant un cathéter allongé (11) ayant des extrémités proximale et distale, un élément d'ancrage auto-extensible (14) ayant des extrémités proximale et distale monté sur une partie distale du cathéter (11), une greffe aortique (12) dans un état comprimé radialement ayant des extrémités proximale et distale et montée sur le cathéter (11) en étant adjacente de manière proximale à l'élément d'ancrage (14), **caractérisé en ce que**
l'extrémité distale de la greffe (12) ne recouvre pas l'extrémité proximale de l'élément d'ancrage (14), et le système d'alimentation en greffe (10) comporte des moyens d'extraction (13) fixés sur l'extrémité distale de la greffe (12), et étant agencés pour passer d'une manière telle à travers le système d'alimentation en greffe (10), que lors d'une traction sur ceux-ci à partir de l'extrémité proximale du système d'alimentation en greffe (10), l'extrémité distale de la greffe (12) est retirée de l'extrémité proximale de l'élément d'ancrage (14).

5. Système d'alimentation en greffe selon la revendication 4, dans lequel l'élément d'ancrage auto-extensible (14) est formé d'un matériau à mémoire de forme.

6. Système d'alimentation en greffe selon la revendication 5, dans lequel l'élément d'ancrage (14) est formé d'un alliage de NiTi.

7. Système d'alimentation en greffe (10) selon l'une quelconque des revendications 1 à 6, dans lequel le cathéter (11) comporte de plus un passage (18) ayant une ouverture (19) située au niveau de l'extrémité distale, et s'étendant vers l'extrémité proximale, et les moyens d'extraction (13) comportent au moins un fil de traction (13) s'étendant depuis l'extrémité distale de la greffe (12) à travers l'ouverture (19) située au niveau de l'extrémité distale du cathéter (11) vers l'extrémité proximale du cathéter (11).

8. Système d'alimentation en greffe (10) selon la revendication 7, dans lequel les fils de traction (13) sont lacés à travers l'élément d'ancrage (14).

9. Système d'alimentation en greffé (10) selon l'uns quelconque des revendications 1 à 8, dans lequel le système d'alimentation en greffe (10) a un diamètre inférieur à environ 7 mm.

10. Système d'alimentation en greffe (10) selon l'une quelconque des revendications 1 à 9, dans lequel l'élément d'ancrage (14) comporte un extenseur (14).

11. Système d'alimentation en greffe (10) selon l'une quelconque des revendications 1 à 10, dans lequel la greffe (12) comporte de plus des moyens de guidage (27) s'étendent à partir de l'extrémité proximale de la greffe (12).

12. Système d'alimentation en greffe (10) selon la revendication 11, dans lequel les moyens de guidage (27) comportent au moins un fil de guidage (27).

13. Système d'alimentation en greffe (10) selon la revendication 7, comportant un cathéter (26) pour disposer des moyens de fixation (25) sur le au moins un fil de traction pour fixer la greffe (12) sur l'élément d'ancrage (14).

14. Système d'alimentation en greffe (10) selon l'une quelconque des revendications 1 à 13, dans lequel la greffe (12) est une greffe à ramifications (12).
